# EUROPEAN PATENT APPLICATION

(11) **EP 4 167 240 A1**
(43) Date of publication of application: **19.04.2023**
(21) Application number: 21207897.6
(22) Date of filing: 12.11.2021
(51) Int. Cl.: G16H 10/60, G16H 30/20, G16H 30/40, G06F 16/20, G06F 16/50

(54) **SYSTEM AND METHOD FOR ULTRASOUND IMAGE HANDLING**

(30) Priority: 14.10.2021 US 202163255628 P
(71) Applicant: Koninklijke Philips N.V., 5656 AG Eindhoven (NL)
(72) Inventor: SUTTON, Jonathan Thomas, Eindhoven (NL); ADDEPALLI, Jeevan Ram, Eindhoven (NL); BHARAT, Shyam, Eindhoven (NL); KUMARESAN, Niranjan Babu, Eindhoven (NL); SK, Santhosh, Eindhoven (NL); BRADLEY, Kevin, Eindhoven (NL); GREINER, Harald, Eindhoven (NL)
(74) Representative: Philips Intellectual Property & Standards

(57) **Abstract**

A method for enabling distributed compilation of a multi-layered ultrasound data record for a patient, through a system having multiple input devices at different locations, each having access to a database, and each able to contribute to an ultrasound data record for a patient in accordance with a certain privilege or access status.

## Description

### FIELD OF THE INVENTION

This invention relates to a system and method for use in a clinical workflow involving ultrasound image acquisition and analysis.

### BACKGROUND OF THE INVENTION

Over the past decade, tools for providing absolute measures of central hemodynamics in an intensive care unit (ICU) environment have fallen out of favor due to associated morbidity and questionable accuracy. For example, the Swan-Ganz catheter, once regarded as the gold-standard tool for monitoring cardiac output and pulmonary pressures, has been phased out in Europe and is used mostly by cardiac care units in North America. Consequently, ICU care providers have begun to use more non-invasive tools to measure the same parameters, such as bioimpedance, single element transesophageal Doppler flow, and pulse contour analysis.

Though these tools reduce the complication rate compared to invasive approaches, the hemodynamic measures provided to the clinician are relative and unstable.

Ultrasound imaging has long been used as a diagnostic tool in clinical environments due to its direct assessment of anatomy. However, its widespread use has been limited by the requirement for expert operators, thus significantly restricting its use to scheduled diagnostic examinations by radiology or cardiology teams. This requirement is particularly difficult to manage in the setting of intensive care, where patients at risk of cardiac decompensation can deteriorate rapidly, necessitating ad hoc, rapid assessments of the heart. Further, ultrasound data obtained in the critical care environment is often only stored in a local ICU database, with the majority of images not forwarded to institutional PACS, or shared among the critical care team.

Recent advances in ultrasound technology such as hardware miniaturization, ultrasound digitation, and cost reduction have made it much easier for care providers in an ICU environment to access devices and achieve sufficient competency in ultrasound for quick assessments. Ongoing development of machine-learning based image analysis tools will continue to facilitate use of ultrasound within the ICU environment, making it easier for experts and novice users to acquire images of sufficient quality to manage their patients at the point of care.

### SUMMARY OF THE INVENTION

Despite the advancements outlined above, the interaction between expert and non-expert, as well as the manner in which ultrasound image data are quantified and handled by multiple users of various skill levels or expertise remain a potential source of confusion and/or clinical errors.

There is therefore a need for a system supporting such interaction in a distributed manner, with which different users may interact to perform a plurality of operations in relation to an ultrasound dataset for a patient.

The invention is defined by the claims.

According to examples in accordance with an aspect of the invention, there is provided a method for implementation by a distributed computing system.

The aforementioned distributed computing system includes a patient datastore storing ultrasound data records for a plurality of patients, each ultrasound data record including ultrasound data for a respective one of the plurality of patients.

The method comprises receiving at an ultrasound imaging apparatus patient identification data.

The method further comprises receiving at a server ultrasound imaging data of the patient from the ultrasound imaging apparatus.

The method further comprises storing the ultrasound imaging data in an ultrasound data record for the patient in the patient datastore, tagged with the patient identification data.

The method further comprises receiving at the server a datastore access request from at least a first client device, the access request including identification data of a patient to whose record access is requested, and user authentication data for authenticating the user requesting access to the datastore.

The method further comprises receiving supplementary data from the first client device for appending to the patient ultrasound data record, and appending the supplementary data to the data record to result in a supplemented patient ultrasound data record.

The method may further comprise accessing the supplemented patient ultrasound data record at a further device. This may for example be done at a patient monitor subsystem. It may be done at a further client device. It may be done at the ultrasound imaging apparatus.

Thus, embodiments of the invention provide a distributed system permitting generation of a comprehensive patient ultrasound data record through enabling multiple different users to access, edit and add to the ultrasound record from different locations. This is mediated through a common identifier in the form of a patient ID, which permits multiple users (e.g. with different specialties, and different skill levels) at multiple locations to contribute to a patient record. It has not before been considered to provide a system which permits acquired ultrasound data to be directly accessed and supplemented (e.g. with annotation or clinical review) by users outside of the direct patient monitoring environment. This accelerates diagnostic assessment, and improves patient outcome, since it enables different aspects of an ultrasound scan (image acquisition, quantification, review, diagnostic analysis) to be performed in a distributed manner by practitioners in multiple physical locations.

The patient ID data is for identifying a patient who is to be imaged using the ultrasound imaging apparatus. This may be received at the ultrasound imaging apparatus locally (e.g. input by an operator) or may be received from a network connection, e.g. from a patient monitoring subsystem.

The aforementioned server may be a single computing device, or may be a distributed server subsystem, for example cloud-based, and accessible by a particular network address.

The user authentication data may include user identification data, and secure authentication data, for use in verifying the user's identity.

The server may act to police access to the patient datastore, by performing verification of user authentication data received in each datastore access request. For example, the datastore access request may be received at the server. The server may perform verification of the permissions status of the user. Depending upon the result of the verification (positive or negative), access may be granted to the requested data record or not. If access is granted, the server may further receive the supplementary data from the first client device and append it to the relevant patient data record.

As mentioned, the method may comprise a step of verifying a permissions status of the user in relation to the requested patient data record based on the received authentication data, and, dependent upon the permissions status, grant access to the requested patient record (or deny access).

In some embodiments, the system includes a permissions status datastore recording a permissions status for each of a plurality of users in relation to each of one or more patients.

In some examples, the permissions status defines a permissions level along a graded scale which differentially grants a user one or more of the following permissions:
permission to access a patient's ultrasound data record;
permission to add annotation data to a patient ultrasound data record; and
permission to verify one or more existing annotations to a patient's ultrasound data record.

As mentioned above, in some examples, the system includes a patient monitor subsystem. The patient monitor subsystem may include at least one patient monitor device, e.g. a bedside patient monitor device. The patient monitor subsystem may include at least one user interface. The previously mentioned supplemented patient ultrasound data record may be accessed at the patient monitor subsystem.

Additionally or alternatively, the supplemented patient ultrasound data record may be accessed by the ultrasound imaging apparatus and displayed on a user interface of the ultrasound imaging apparatus.

The system may in some examples include a dedicated interface unit connected between the ultrasound imaging device and a patient monitor subsystem comprised by the system, and wherein the supplemented patient ultrasound data record is accessed at the interface device. For example, the interface unit may be a bridge unit which connects to the ultrasound imaging apparatus and the patient monitor device.

In some examples, the method may further comprise controlling a user interface of the patient monitor subsystem, to display the ultrasound data and the supplementary data.

In some embodiments, the method may further comprise pushing an ultrasound data record to a specific user device, and prompting the user for input. For example, the method may comprise one user generating at one client device a review request message, the review request message including a pointer to a specific patient ultrasound data record in the patient datastore, and including identification information of a (different) target recipient user for the message. The method may further comprise porting or routing the message from the transmitting client device to the recipient client device.

The aforementioned appended supplementary data may be tagged with user identification data identifying the relevant user as the author of the supplementary data.

The supplementary data may, by way of non-exhaustive example, comprise: annotation of at least one ultrasound image; a linguistic message; and/or an approval indicator, indicating approval or non-approval of annotation data included in the ultrasound imaging data.

The linguistic message may contain diagnostic information, or other analysis of the ultrasound image data.

The annotation data which is the subject of the approval or non-approval may be quantification data, for example one or more computed estimates of hemodynamic parameters such as cardiac output or stroke volume.

In some examples, the method may further comprise establishing a live two-way communication channel between at least one client device and the ultrasound imaging apparatus. The method may comprise streaming user-input feedback from the at least one client device to a user interface of the ultrasound imaging apparatus. Additionally or alternatively, the method may comprise steaming real-time ultrasound acquisition data from the ultrasound imaging apparatus to the at least one client device.

Where the system includes an interface device connected between the ultrasound imaging device and the patient monitor subsystem, the communication channel may pass via the interface unit.

The establishing of the aforementioned communication channel may be performed dependent upon verifying a permissions status of a user logged in to the at least one client device.

With reference to the previously discussed client device, this may be a mobile computing device.

A further aspect of the invention provides a server for a system, comprising:
a communication unit for communication with
   a patient datastore storing ultrasound data records for a plurality of patients, each ultrasound data record including ultrasound data for the patient, and
   one or more client devices of the system; and
one or more processors adapted to:
   receive at the communication unit from at least a first client device a datastore access request, the access request including patient identification data of a patient to whose record access is requested, and user authentication data for authenticating the user requesting access to the datastore; and
   receive supplementary data from the first client device for appending to the patient ultrasound data record, and appending the supplementary data to the data record to result in a supplemented patient ultrasound data record.

The one or more processors of the server may be further adapted to:
receive ultrasound imaging data for a patient from an ultrasound imaging apparatus; and
store the ultrasound imaging data in an ultrasound data record for the patient in the patient datastore, tagged with patient identification data.

The one or more processors of the server may further be adapted to verify a permissions status of the user in relation to the requested patient data record based on the authentication data, and, dependent upon the permissions status, grant access to the requested patient record.

A further aspect of the invention provides a distributed computing system, comprising:
a patient datastore for storing ultrasound data records for a plurality of patients, each ultrasound data record including ultrasound data for the patient;
one or more client devices; and
a server module in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a computing system and dataflow in an example method according to one or more embodiments;
Fig. 2 illustrates an example computing system according to one or more embodiments;
Fig. 3 illustrates data access and data input at a client device of the system;
Fig. 4 illustrates an example network environment within which the system may operate in some examples; and
Fig. 5 further illustrates the example network environment in which the system may operate.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for enabling distributed compilation of a multi-layered ultrasound data record for a patient, through a system having multiple input devices at different locations, each having access to a database, and each able to contribute to an ultrasound data record for a patient in accordance with a certain privilege or access status. For example, different ultrasound users may have access to an ultrasound image for manipulation (e.g. interpretation, quantification, annotation, etc.) before or while the ultrasound image is displayed on a patient monitor, for example together with other physiological parameters of the patient.

Embodiments may therefore comprise a processor (e.g. a server or bridge module) in two-way communication with one or more of: (i) an ultrasound imaging apparatus, (ii) a patient monitor unit and (iii) one or more client computing devices. The processor is configured to receive a plurality of data (such as images and/or computed physiological parameter(s)) and transmit a plurality of data (such as images and/or physiological parameter(s))). The processor may be further configured to receive instruction signals from one or more client computing devices and process instructions of the instruction signals such as to send one or more instruction signals to either or both of (i) the ultrasound apparatus and (ii) patient monitor unit.

In a preferred set of embodiments, the one or more client computing devices are connected to a user account which enables the user to access a pre-determined amount of pre-set functionalities (more or less), thereby facilitating different levels of instructions via the instruction signals based on the user's need/credentials.

Embodiments therefore provide a computer-implemented method (e.g. an application for installation on a computing device) configured to perform one or more of the following functions, each of which will be further explained in more detail to follow:
enable interaction across a clinical team through a patient monitor user interface;
display on such a user interface ultrasound data together with quantification data derived from the ultrasound data (live or pre-acquired);
communicate with one or more databases, wherein the databases comprise quantification and imagespecific information that are retrievable based on user credentials;
output ultrasound data overlaid with retrieved information from the one or more database on the first and/or a second user interface.

The integration of ultrasound as a modality into patient monitoring creates opportunities for expanded use of the technology by different care providers and in new clinical scenarios, e.g. ICU. However, there remains a need for a means to facilitate quantification and analysis of ultrasound data by multiple users of various skill levels or expertise. For example, it is valuable to enable a less skilled sonographer to perform quick periodic cardiac ultrasound scans, at the patient bedside, and have these assessed by an expert for quantification review and diagnostic decision making, although the expert may not be physically present. A solution is needed which bridges this communication gap. Embodiments of the present invention provide a system and method to enable these interactions in a distributed manner, by creating a distributed application with which credentialed users interact to perform operations such as automated interpretation approval, quantification editing, annotation, and education.

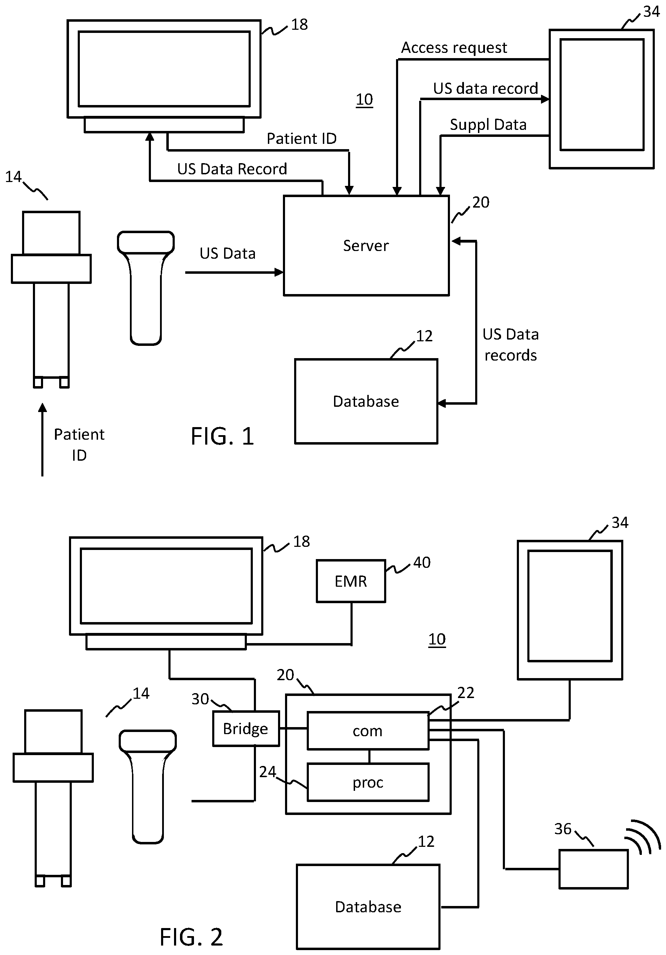

An example system is illustrated in Fig. 1, along with an example dataflow among components of the system.

The system 10 includes an ultrasound imaging apparatus 14 for acquiring ultrasound data of a patient. For example, this may be a cart-based ultrasound apparatus, which may for example be used at a patient's bedside. The ultrasound imaging apparatus may comprise a hand-operated probe or may comprise a body-mountable ultrasound transducer unit, e.g. a patch, for monitoring purposes. The ultrasound data may be cardiac ultrasound data in some examples.

In some examples, the ultrasound imaging apparatus 14 includes physiological parameter quantification functionality. For example, it may be operable to compute, based on processing of acquired cardiac ultrasound data, one or more hemodynamic parameters of the subject.

The illustrated example includes a central server 20 which acts as interface between components of the distributed system 10. The steps of the method will be explained with reference to such a server. However, in practice, there need not be any single central server module which mediates the method. Each individual node device which is comprised by the system may individually communicate with a joint database to upload or download data. Each device may directly communicate with other devices. In some examples, communication among the nodes of the system may be facilitated by a cloud-based communication system, i.e. via an internet-based portal.

The system includes a datastore storing a database 12, the database containing ultrasound data records for a plurality of patients. This may in some cases be comprised by the central server 20. In some examples, the server might be a server of an institutional PACS (Picture Archiving and Communication System) of the hospital in which the system is being implemented. In other examples, the datastore could be a local datastore of the patient monitoring environment.

The system preferably further includes a patient monitoring subsystem 18, which includes one or more patient monitoring devices, each comprising a user interface including a display. The patient monitoring subsystem 18 may include at least one bedside patient monitor device. It may include a central patient monitor console for connection to a plurality of bedside patient monitor devices.

The system further includes one or more client devices 34 adapted to communicate with the server 20 to access and add to ultrasound data records stored on the database 12. One or more of the client devices may be mobile computing devices, such as smartphones or tablet computers.

In summary, a dataflow of the system may be as follows.

Before acquisition of ultrasound data, patient ID information is input to the ultrasound imaging apparatus 14. This may be performed manually using a keypad or touchpad. It may be performed through scanning a barcode or QR code. It may be achieved through scanning an NFC tag. In some examples, the patient ID data may be ported to the ultrasound imaging apparatus from the patient monitoring subsystem 18.

Ultrasound imaging data is then acquired. This data is then transmitted to the server 20, along with the patient ID information, e.g. in the form of metadata, or tag data. The sever module 20 may then transfer the ultrasound imaging data to the database 12, tagged or otherwise linked with the patient ID information. For example, it may be stored in an ultrasound data record for the patient.

The ultrasound imaging data may be communicated to the server 20 in real time with acquisition. Alternatively, it may be communicated as a single combined data package to the server after an extended period of acquisition.

The patient monitoring subsystem 18 may communicate with the server 20 to access ultrasound data records for one or more patients. It may communicate relevant patient ID information to the server 20, and the server may port to the patient monitoring subsystem data from the ultrasound data record(s) of the relevant patient from the database 12.

Fig. 1 shows a first client device 34. The system may include a greater number of client devices. This may be a mobile computing device such as a smartphone or a tablet or may be a computer console or any other type of computing device. The client device communicates to the server a datastore access request. The access request may include identification data of a patient to whose record access is requested, and user authentication data for authenticating the user requesting access to the datastore. The authentication data may include user identification information, and user authentication secure data such as a password or biometric data.

The server 20 performs verification of a permissions status of the user in relation to the requested patient data record based on the authentication data. Dependent upon the permissions status, access is granted to the requested patient record or access is denied. Access may be granted at a certain permissions level. The system may include a permissions status database recording a permissions status for each of a plurality of users in relation to each of one or more patients. The permissions status may define a permissions level along a graded scale which differentially grants a user one or more of the following permissions: permission to access a patient's ultrasound data record; permission to add annotation data to a patient ultrasound data record; and permission to verify one or more existing annotations to a patient's ultrasound data record.

The user of the client device 34, if access to the patient data record is granted, may submit various types of supplementary data. These may include one or more of:
annotation of at least one ultrasound image;
a linguistic message;
an approval indicator, indicating approval or non-approval of annotation data included in the ultrasound imaging data.

Fig. 2 shows in more detail the architecture of an example system which may be utilized according to one or more embodiments. As shown, the system 10 includes the ultrasound imaging apparatus 14, the server 20, the database 12, the patient monitoring subsystem 18 and the at least one client device 34 previously discussed. As shown in Fig. 2, the server may include a communication module 22 such as an input/output, and a coupled one or more processors 24 connected to the communication module. In this illustrated example, the system further includes an interface or bridge unit 30 which is connected operatively between the ultrasound imaging apparatus 14 and the patient monitoring subsystem 18. This may facilitate direct communication between the patient monitor and the ultrasound imaging apparatus. The patient monitoring subsystem may be operatively connected to an electronic medical record (EMR) system 40. The patient monitoring subsystem may receive ultrasound data from the ultrasound imaging apparatus via the bridge unit 30. In some examples, one of the bridge unit or the patient monitor subsystem 18 itself may compute one or more physiological parameters from the ultrasound data. For example, hemodynamic parameters such as cardiac output, stroke volume, ejection fraction, end diastolic/systolic volume, and/or global longitudinal strain may be computed from cardiac ultrasound data using suitable quantification algorithms. Quantification algorithms may employ use of automated segmentation algorithms.

The system may further include one or more additional network connections 36 for connection to further patient monitoring or patient data storage systems.

One aspect of the invention may be a computer program product for execution by a respective processor of each client device 34 and/or each node device of the whole system (e.g. including the ultrasound imaging apparatus 14 and/or the patient monitor 18). The computer program, when run on a processor of a device, may provide a graphical user interface to the user, and facilitate the transmission of data access request messages, receipt of review request messages, the input of supplementary data, and the transmission of the supplementary data to the server. The computer program, when executed on a device, may control the device to prompt the user to log in by inputting user credentials, including user identification information and user authentication information. The computer program may query a user credentials database. The computer program, following successful login by verifying the user credentials, may provide the user a graphical interface which permits the user to perform a selection of actions, wherein the available actions may vary depending upon a permissions status of the user. The user may input patient ID information, and trigger transmission of a data access request message to the server, requesting access to the relevant patient data record. The user be presented with a list of received review request messages, and options for providing input.

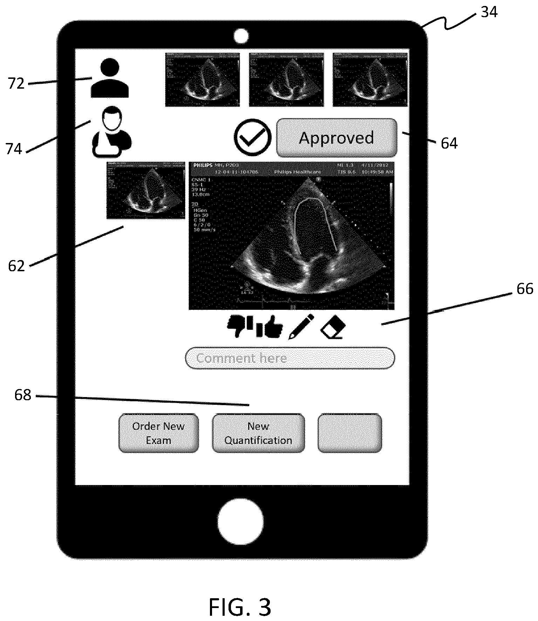

Fig. 3 provides an illustration of one or more functions which may be implemented by the at least one client device 34 in accordance with one or more embodiments. The client device in this example is a mobile computing device, e.g. a tablet computer or smartphone, and the user interface comprised by the client device is a touch-screen display.

In some examples, the user interface of the client device may display 62 one or more ultrasound images obtained at the ultrasound imaging apparatus 14. The user interface of the client device may prompt 64 the user for input such as approval of obtained images, or approval of physiological parameter quantifications. The user may be prompted to select one of a set of images as a best image. The user may be prompted to approve certain segmentation or annotation data which has been appended to one or more images, either automatically or by another user. The user may be prompted to edit or add annotation data 66 to one or more images. The annotation data may include outlines of one or more anatomical structures, or may be textual comments for adding to an image. In some examples, the user may be provided selectable options 68 to order new examinations to be performed on a patient, or new physiological parameter quantifications to be performed based on the imaging data.

The user may log into a user account 72 access portal on the server module 20 using user credentials. The user may request access to data for any of a range of patients 74 to whose records their permissions status permits them access.

A number of optional further features will now be outlined.

In some embodiments, the system 10 may include functionality for establishing a live two-way communication channel between at least one client device 34 and the ultrasound imaging apparatus 14.

This communication channel may be used for example to provide streaming of user-input feedback from the at least one client device to a user interface of the ultrasound imaging apparatus. Additionally or alternatively, it may be used to provide steaming of real-time ultrasound acquisition data from the ultrasound imaging apparatus to the at least one client device.

Thus, embodiments may enable a "live" functionality to enable remote expert input during an ultrasound scan. For example, if a novice user is having trouble with an acquisition, an expert user located elsewhere can connect to the live ultrasound image stream from their client device. For example, the client device may execute an application which connects to the server using the user credentials and based on the patient ID. In some examples, the ultrasound operator at the patient bedside may transmit a call request to the remote expert user which causes their client device to issue an alert and permits direct connection into a two-way communication channel with the ultrasound imaging apparatus, via the server module 20.

Once connected, the communication channel shows the live ultrasound images streamed from the current scan. At the patient bedside, the novice user also operates a further instance of the computer-implemented application. This may be run on the ultrasound imaging apparatus, or on a separate client device. This may show the live ultrasound images. It may further provide live feedback from the expert user. This live feedback might take various forms, e.g. auditory, video, textual (e.g. automatically transcribed text on screen).

The expert user may, using their client device, select one of a set of previously acquired images from a prior patient history which represents a target view of an anatomical structure which the novice user may seek to emulate.

Thus, according to this embodiment, both the novice user at the bedside and the expert user may be simultaneously running on a local device a patient-specific instance of a computer-implemented application or method which provides connection with the server 20, and which provides a graphical user interface on the device by which the user may control execution of the various functions discussed above. The application may have a user login function which permits a user to connect to the server with their user ID information and user authentication information, and depending upon a set of permissions which are associated with the user in a permissions database, the graphical user interface presented to the user may provide the user with a different set of performable actions. Thus, the screen presented to the user is customizable depending upon user permissions in this case. For example, it may permit them to add different types of annotation data and to perform different approval or verification actions in relation to image data and annotation data added by other users.

According to one or more embodiments, if an expert user is not immediately available during an ultrasound image acquisition session, the system may include means to permit the expert user to add their input at a subsequent time point. For example, the system may provide functionality for generating push messages which are transmitted to relevant users to prompt them to provide input on one or more ultrasound image data sets.

For example, the system may provide functionality for generating at a client device of a one user (e.g. a novice user) a review request message, the review request message including a pointer to a specific patient ultrasound data record in the patient datastore, and including identification information of a target recipient user for the message. The target recipient user is the user to whom the message is to be directed. This metadata allows for porting of the message from the client device to a further client device, e.g. belonging to an expert user.

By way of example, a novice user may perform a new ultrasound data acquisition. They require expert input by a further clinician. This input, by way of example only, may be to check the quality of the images acquired, to provide diagnostic analysis, to select one of a set of images, or to approve one or more physiological parameter quantifications. The novice user controls generation of an input-request message on the user interface of their client device. This includes input of a target second user for receipt of the message. When the message is sent, patient ID information is included and a pointer to a particular ultrasound data record for the patient in relation to which input is desired. This patient ID information may be already stored in a local cache on the first client device,

When the message is sent, it is routed, for example via the server 20, to the client device of the expert user. An alert may be generated on the expert user's client device, who can then review the acquired images and provide input. By way of example, if any changes are needed to the acquired images or to quantification data or diagnostic data added by the novice user, this can be input. A return message may then be ported to the novice user's client device indicating the provided input by the expert, including any suggested changes or additional images to acquire. Thus, this two-way communication pathway enables quick feedback and correctional action, without needing to wait for a physical visit by the expert clinician to the patient bedside.

The described method and system permits a plurality of client devices to communicate in a linked network arrangement to input various contributions to a patient ultrasound data record, through use of a patient identifier (ID) to link each contribution to the same common patient data record.

This concept may be implemented as part of a broader system in which the whole patient journey including admission, examination with imaging, and review by clinicians is performed through use of the common patient identifier to link each event to the patient record.

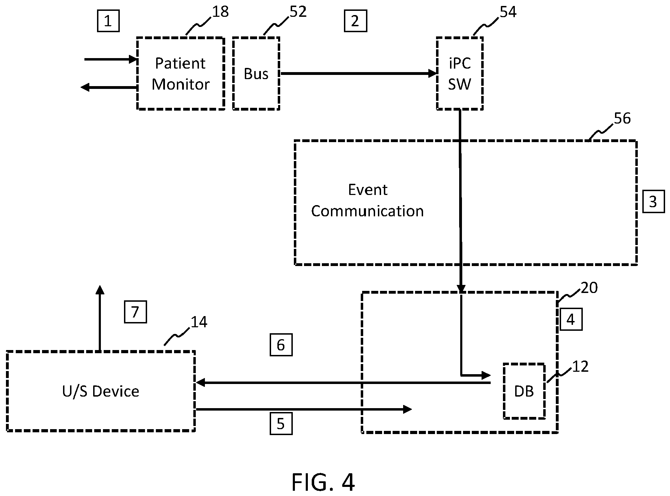

An example architecture of such a broader system will now be outlined by way of illustration, with reference to Fig. 4 which shows the system and an example event process flow.

The system includes a patient monitoring subsystem 18 which includes one or more patient monitor consoles. Each patient monitor console houses a co-resident personal computer 54 (iPC) for facilitating messaging and communication with a processing server 20. This server 20 provides the server 20 referred to previously with reference to Figs. 1 and 2.

The system processing server 20 may in simple examples be a personal computer running a standard proprietary operating system. This may be configured to implement (i) a Modality Worklist (MWL) and Modality Procedure Performed Step (MPPS) server and a lightweight database 12 which may hold the patient data records, (ii) a messaging broker which enables secure communication between the MWL/MPPS servers and the one or more patient monitors 18, and (iii) a DICOM storage server. The terminology used above, in particular, MWL and MPPS, are standard DICOM terms, and will be familiar to the skilled person in this field. For example, an MWL server is a server that stores a list of requested procedures to be performed on one or more different medical imaging devices. An MPPS server facilitates MPPS messages from imaging devices indicative of what acquisition steps have been performed.

The system may optionally further include a Bus 52 for providing communication with a further external system or server, for example an electronic medical record (EMR) system. This Bus may permit for example access to the system by third parties. For instance, data acquired at the patient monitor may be directly accessed by certain further subsystems through this Bus or data may be transmitted to the patient monitor via this Bus.

The system further includes one or more ultrasound imaging devices 14, which are configured to communicate with the above-mentioned MWL and MPPS servers 20.

By way of one example, the steps and flow of data to initiate an ultrasound scan (termed an ultrasound study) on an ultrasound imaging device may be as follows.

The process starts (step 1) with patient admission. This involves as event at the patient monitor 18. There is input a change in the admission status on the patient monitor. This modifies internal memory information within the patient monitor, and allows new vital signs information to be collected for a patient. This event further triggers retrieval of information about the admitted patient (e.g. patient medical record number) from the care provider or the patient information center.

At step 2, an event is triggered within the system as a result of the change in the admission status of the patient.

In step 3, the triggered event causes an ADT event message to be generated 56 and communicated to the processing server 20: The ADT event contents are transmitted to the server via a JSON message containing necessary event description information such as Patient Medical Record Number (MRN), Age, Weight, and timestamps.

At step 4, the processing server 20 receives the message and stores new patient information in the database 12. This database 12 provides the patient datastore referred to previously with reference to Fig. 1 and Fig. 2.

One new study entry in the database 12 for each patient is always maintained. A study entry means a data entry associated with the patient, and associated with a particular instance of a medical imaging examination (i.e. a study). In the described system, it is an aim to allow imaging procedures to be performed ad hoc at the patient bedside, and stored in the database 12. By always keeping a study entry open, this allows this to be achieved. By contrast, more typically in a DICOM system, a clinician "orders" an exam to be conducted on a patient, this being facilitated at the MWL server, which results in one study entry being created on the database for the ordered study. In the present system however, the source of the new worklist entry is not a clinician but rather a patient admission event on a patient monitor. The processor 20 ensures there is a new, open MWL entry in the server that corresponds to that patient. This allows an operator to visit the bedside in an ad hoc fashion and always have an entry waiting to be fulfilled.

At the ultrasound device, when a care provider needs to perform a patient scan, an MWL query is sent (step 5) to the processing server from the ultrasound device 14 with the patient ID information for the patient for which a scan is to be performed.

The server 20 responds (step 6) to the query with an MWL entry containing the necessary information to complete a scan for that patient (e.g. study instance unique identifier (UID), age and/or weight if necessary, last name if necessary, patient MRN).

The care provider may then initiate the ultrasound examination at the ultrasound device 14 (step 7).

The server 20 is also adapted to perform the functions described previously in this disclosure of facilitating access to the database 12 by one or more client devices 34 and updating the database with supplementary data received from the client device. The server 20 may include a communication module or Bus to facilitate this (not shown).

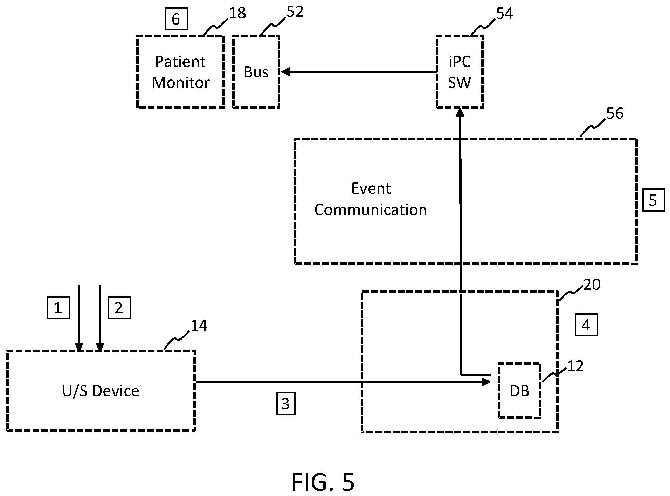

Fig. 5 schematically illustrates the data flow in the reverse direction, during or following an ultrasound examination, for appending the ultrasound data to the database and porting the results to the patient monitor and/or other devices.

The process comprises (step 1) ultrasound data acquisition at the ultrasound imaging device. The process may further comprise (step 2) image quantification performed by an operator of the ultrasound imaging device. Quantification involves the care provider either manually, or though execution of one or more automated physiological parameter quantification algorithms, generating values for one or more physiological parameters. These may for example include one or more hemodynamic parameters such as cardiac output and stroke volume.

The process further comprises exporting (step 3) the ultrasound imaging data and the generated quantifications (i.e. the study results) along with a compiled DICOM Structured Report (SR) to the processing server 20 for storage on the database 12, where the database acts a pre-configured DICOM storage node. A DICOM SR is type of DICOM file which encapsulates generated numbers and notes input by a user at step 2 from reviewing the image.

The process further comprises (step 4) the processing server 20 storing the study results as DICOM files in the database 12. This includes the acquired image data as well as the generated structured report previously mentioned.

Based on a lookup table which records the particular patient monitor label associated with each specific DICOM SR, the server 20 recognizes DICOM metadata tags in SR files, extracting them from the file, and storing the data in the database associated with the correct patient.

The process further comprises pushing (step 5) the study results to the patient monitor 18, via the personal computer 54 associated with the patient monitor. This comprises generating a data package containing the numeric results (quantifications) as well as the imaging data. This is sent as a message over a secure message transfer network 56 to the patient monitor 18.

The patient monitor 18 receives (step 6) the study results and may display these on a user interface. The results may be forwarded to a central patient monitoring unit.

In some embodiments, the processing server 20 may be adapted to communicate with a further patient information center. The patient information center may be arranged to receive all acquired data for all patients in an institution from different clinical areas and imaging modalities and to store the data and/or to route the data to corresponding patient monitor devices. For example, the patient information center may act to forward the ultrasound results to a specific patient monitor over a network connection, for example to a patient monitor which has been initiated with the corresponding patient ID information.

In one or more embodiments, the one or more patient monitor units 18 may be adapted to connect to a web server and to receive ultrasound imaging data pertaining to the patient being monitored by the monitor unit. This imaging data may be sourced from the DICOM storage node co-resident with the processing server unit, or from a DICOM storage server facilitated by a central institutional PACS (Picture Archiving and Communication System) server.

As mentioned above, the patient identifier information may be input to each device or node of the system before events are executed in relation to a particular patient. For example, patient ID information may be input to the ultrasound device before ultrasound data is acquired so that the data can be automatically linked to the patient's data record on the database 12. Likewise, patient ID information is input to the patient monitor, so that the patient monitor can access relevant patient ultrasound image data and quantifications from the server.

The patient identifier information may be input manually by a user at a given device or may be transferred automatically, e.g. using an optically scannable code, or an RFID tag. In some examples, patient identifier information may be transferred via Bluetooth using Bluetooth proximity technology. By way of one advantageous example, an ultrasound imaging device may obtain patient identifier information from a nearby patient monitor through Bluetooth. When an ultrasound imaging device is wheeled to the patient bedside to perform an examination, the ultrasound device may recognize patient monitors that are in close proximity and retrieve patient information from a closest of those patient monitors. In some examples, a shortlist of patients may be presented to the clinician and the clinician selects one from the shortlist, and proceeds with the examination.

Various embodiments outlined above make reference to use of a server. In each case, it is to be understood that the server may be a single, unitary device comprising one or more processors, or may be a distributed server, meaning that the server may be implemented by a network or system of processing devices which are distributed across multiple locations. The server may in some examples be a cloud-based server. The server may be an internet-based sever. The server may be facilitated by an internet portal. Therefore, the particular physical hardware configuration of the server is not essential.

As discussed above, embodiments of the system make use of a processor. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for implementation by a distributed computing system (10),
wherein the computing system includes a patient datastore (12) storing ultrasound data records for a plurality of patients, each ultrasound data record including ultrasound data for a patient of the plurality of patients;
wherein the method comprises:
receiving at an ultrasound imaging apparatus (14) patient identification data;
receiving at a server (20) ultrasound imaging data of the patient from the ultrasound imaging apparatus;
storing the ultrasound imaging data in an ultrasound data record for the patient in the patient datastore, tagged with the patient identification data;
receiving at the server (20) a datastore access request from at least a first client device (34), the access request including patient identification data of a patient to whose record access is requested, and user authentication data for authenticating the user requesting access to the datastore;
receiving at the server supplementary data from the first client device for appending to the patient ultrasound data record, and appending the supplementary data to the data record to result in a supplemented patient ultrasound data record; and
accessing at a further device the supplemented patient ultrasound data record.

2. The method of claim 1, further comprising the server verifying a permissions status of the user in relation to the requested patient ultrasound data record based on the authentication data, and, dependent upon the permissions status, granting access to the requested ultrasound data record.

3. The method of claim 1 or 2, wherein the system includes a patient monitor subsystem (18), the patient monitor subsystem including at least one user interface, and wherein the supplemented patient ultrasound data record is accessed at the patient monitor subsystem, and
optionally wherein the method further comprises displaying on a user interface of the patient monitor subsystem (18), the ultrasound imaging data and the supplementary data.

4. The method of any of claims 1-3, wherein the system includes an interface device (30) connected between the ultrasound imaging device (14) and a patient monitor subsystem (18), and wherein the supplemented patient ultrasound data record is accessed at the interface device.

5. The method of any of claims 1-4, wherein the method comprises:
generating at a further client device a review request message, the review request message including a pointer to a specific patient ultrasound data record in the patient datastore, and including user identification information of a target recipient user for the message; and
porting the message from the further client device to the first client device.

6. The method of any of claims 1-5, wherein the supplementary data comprises one or more of:
annotation of at least one ultrasound image;
a linguistic message;
an approval indicator, indicating approval or non-approval of annotation data included in the ultrasound imaging data.

7. The method of any of claims 1-6, wherein the system includes a permissions status datastore recording a permissions status for each of a plurality of users in relation to each of one or more patients.

8. The method of claim 7, wherein the permissions status for each user defines a permissions level along a graded scale which differentially grants a user one or more of the following permissions:
permission to access a patient's ultrasound data record;
permission to add annotation data to a patient ultrasound data record; and
permission to verify one or more existing annotations to a patient's ultrasound data record.

9. The method of any of claims 1-8, wherein the method further comprises establishing a live two-way communication channel between at least one client device (34) and the ultrasound imaging apparatus (14).

10. The method of claim 9, further comprising:
streaming user-input feedback from the at least one client device (34) to a user interface of the ultrasound imaging apparatus (14); and/or
streaming real-time ultrasound acquisition data from the ultrasound imaging apparatus to the at least one client device.

11. The method of claim 9 or 10, wherein the establishing the communication channel is performed dependent upon verifying a permissions status of a user logged in to the at least one client device.

12. The method of any of claims 1-11, wherein the at least first client device is a mobile computing device.

13. A server for a system, comprising:
a communication module for communication with
a patient datastore storing ultrasound data records for a plurality of patients, each ultrasound data record including ultrasound data for the patient, and
one or more client devices of the system; and
one or more processors adapted to:
receive at the communication unit from at least a first client device a datastore access request, the access request including patient identification data of a patient to whose record access is requested, and user authentication data for authenticating the user requesting access to the datastore;
receiving supplementary data from the first client device for appending to the patient ultrasound data record, and appending the supplementary data to the ultrasound data record to result in a supplemented patient ultrasound data record.

14. A server as claimed in claim 13, further adapted to
receive ultrasound imaging data for a patient from an ultrasound imaging apparatus; and
store the ultrasound imaging data in an ultrasound data record for the patient in the patient datastore, tagged with patient identification data.

15. A distributed computing system, comprising:
a patient datastore for storing ultrasound data records for a plurality of patients, each ultrasound data record including ultrasound data for the patient;
one or more client devices; and
a server in accordance with claim 13 or 14.
